(19) Europäisches Patentamt
European Patent Office
Office européen des brevets

(11) EP 2 205 202 B1

(12) **EUROPÄISCHE PATENTSCHRIFT**

(45) Veröffentlichungstag und Bekanntmachung des Hinweises auf die Patenterteilung:
**27.07.2011 Patentblatt 2011/30**

(21) Anmeldenummer: 07845196.0

(22) Anmeldetag: **19.09.2007**

(51) Int Cl.:
*A61K 8/37* *(2006.01)* *B01F 17/36* *(2006.01)*
*A61Q 19/00* *(2006.01)*

(86) Internationale Anmeldenummer:
**PCT/EP2007/059902**

(87) Internationale Veröffentlichungsnummer:
**WO 2007/147904 (27.12.2007 Gazette 2007/52)**

(54) **O/W-EMULGATOR, O/W-EMULSIONEN UND VERFAHREN ZU DEREN HERSTELLUNG**

O/W-EMULSIFIER O/W-EMULSIONS AND METHOD FOR PRODUCTION THEREOF

ÉMULSIFIANTS H/E, ÉMULSIONS H/E, ET PROCÉDÉ DE PRODUCTION CORRESPONDANT

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**14.07.2010 Patentblatt 2010/28**

(73) Patentinhaber: **Symrise AG**
**37603 Holzminden (DE)**

(72) Erfinder:
• **OHRMANN, Rolf**
**21255 Tostedt (DE)**
• **ISSLEIB, Martina**
**22955 Hoisdorf (DE)**
• **ENDLEIN, Edgar**
**29559 Wrestedt (DE)**
• **SCHRÖDER, Bernd**
**37603 Holzminden (DE)**

(74) Vertreter: **Eisenführ, Speiser & Partner**
**Am Kaffee-Quartier 3**
**28217 Bremen (DE)**

(56) Entgegenhaltungen:
**EP-A- 0 227 012     DE-A1- 10 308 565**
**DE-A1- 10 353 030**

• **SYMRISE ET AL: "PEG-freier O/W Emulgator aus Kalium Cetylphosphat und Palmglyceriden" RESEARCH DISCLOSURE, MASON PUBLICATIONS, HAMPSHIRE, GB, Bd. 468, Nr. 115, 1. April 2003 (2003-04-01), XP007132557 ISSN: 0374-4353 in der Anmeldung erwähnt**

## Beschreibung

Die vorliegende Erfindung betrifft Öl-in-Wasser-Emulgatoren (O/W-Emulgatoren), Öl-in-Wasser-Emulsionen (O/W-Emulsionen) und Verfahren zu deren Herstellung.

Emulsionen werden üblicherweise aus zwei flüssigen Phasen geformt, die nicht mischbar sind. Bei der Herstellung einer Emulsion wird eine Phase in fein verteilter Form in der anderen Phase dispergiert. Man unterscheidet im Wesentlichen zwei Typen von Emulsionen, nämlich "Wasser-in-Öl-" und "Öl-in-Wasser-"Emulsionen. Bei der Öl-in-Wasser-Emulsion (O/W-Emulsion) stellt das Öl die innere Phase dar, die in der äußeren (Wasser-)Phase dispergiert ist. Die Eigenschaften der entsprechenden Emulsion werden im Wesentlichen durch die äußere Phase bestimmt, so dass sich Wasser-in-Öl-Emulsionen eher wie Öle und Öl-in-Wasser-Emulsionen eher wie wässrige Lösungen verhalten.

Emulsionen, die als anionische Emulgatoren Alkylphosphate, d.h. Phosphorsäureester, mit Alkylresten mit 6 bis 22 C-Atomen enthalten, sind beispielsweise aus EP 1 264 632 bekannt.

O/W-Emulsionen enthaltend Monoalkylphosphatsalze wie z.B. Monocetylphosphat sind in EP 0 227 012 beschrieben.

EP 0 251 249 beschreibt Mischungen umfassend Alkyl-hydroxyalkylorthophosphorsäure-Estergemische und Co-Emulgatoren wie beispielsweise Cetyl-Stearylalkohol.

EP 0 427 411 beschreibt Sonnenschutzformulierungen enthaltend neutralisierte Mono- und/oder Dialkylphosphate und eine Wachskomponente, wobei auch vollständig hydrierte Pflanzenöle genannt werden.

Research Disclosure No. 468115 vom Mai 2003; No. 469, Seiten 641-644 und WO 2004/075868 beschreiben einen O/W-Emulgator enthaltend (a) gehärtete Palmölglyceride und (b) Kalium-Cetylphosphat, wobei der gewichtsbezogene Gehalt an Kalium-Cetylphosphat zumindest 40 Gew.-% beträgt. Eine dort bevorzugte Ausführungsform betrifft einen O/W-Emulgator bestehend aus:

    (a) 30-50 Gew.-% gehärtete Palmölglyceride,
    (b) 40-50 Gew.-% Kalium-Cetylphosphat,
    (c) 8-15 Gew.-% Cetylalkohol,
    (d) 4-8 Gew.-% Wasser und
    (e) 0-18 Gew.-% weitere Zusatzstoffe,

wobei sich die Gewichtsprozentangaben auf die Gesamtmasse des dort beschriebenen O/W-Emulgators beziehen. Der in WO 2004/075868 beschriebene O/W-Emulgator weist insgesamt sehr gute Eigenschaften auf. In einigen Fällen wurde jedoch eine unzureichende Stabilität der einen Emulgator gemäß WO 2004/075868 enthaltenden Emulsionen festgestellt, insbesondere hinsichtlich der Langzeitstabilität solcher Emulsionen, dabei insbesondere bezüglich deren Wärmestabilität, speziell bei Einarbeitung von insbesondere wasserlöslichen) Sonnenschutzfiltern.

Es war die Aufgabe der vorliegenden Erfindung, einen verbesserten O/W-Emulgator anzugeben, insbesondere einen solchen, der eine verbesserte Stabilität der entsprechenden Emulsionen bewirkt, insbesondere hinsichtlich der Langzeitstabilität solcher Emulsionen, besonders hinsichtlich deren Wärmestabilität, speziell von Emulsionen enthaltend wasserlösliche Sonnenschutzfilter.

Die gesuchten Emulgatoren sollten vorzugsweise eine lediglich geringe Einsatzdosierung verlangen, vorzugsweise universell zur Herstellung von Emulsionen mit unterschiedlichen Viskositäten (z.B. Lotionen, Milchen und Cremes) geeignet sein, vorzugsweise in einem breiten pH-Bereich (von z.B. pH 3 bis 11) angewendet werden können und vorzugsweise eine exzellente Hautverträglichkeit aufweisen. Bevorzugt sollten diese Emulgatoren zudem frei von Polyethylenglycol (PEG) sein.

Ferner sollten O/W-Emulsionen umfassend erfindungsgemäße O/W-Emulgatoren und Verfahren zu deren Herstellung angegeben werden.

Die vorliegende Erfindung betrifft entsprechend einen Öl-in-Wasser-Emulgator (O/W-Emulgator), der hervorragend zur Herstellung von O/W-Emulsionen mit verbesserter Stabilität geeignet ist und der zudem in bevorzugten Ausgestaltungen frei von Polyethylenglycol (PEG) ist.

Gemäß einem ersten Aspekt der vorliegenden Erfindung wird die gestellte Aufgabe gelöst durch einen O/W-Emulgator, umfassend:

    (a) 30 - 50 Gew.-% gehärtete Palmölglyceride,
    (b) 15 - 35 Gew.-% Kalium-Cetylphosphate (Kalium-Monocetylphosphat; Kalium-Dicetylphosphat),
    (c) 20 - 30 Gew.-% Cetylalkohol, und
    (d) 5 - 15 Gew.-% Kaliumphosphate,

wobei sich die Gewichtsprozentangaben auf die Gesamtmasse des O/W-Emulgators beziehen.

Kalium-Monocetylphosphat (CAS-No.: 19035-79-1; INCI: Potassium Cetyl Phosphate) kann auch als Monokaliumsalz des Phosphorsäuremonohexadecylesters bezeichnet werden. Kalium-Monocetylphosphat ist beispielsweise unter dem Namen Amphisol® K kommerziell verfügbar (DSM).

Ein erfindungsgemäßer Emulgator umfasst neben (b) Kalium-Cetylphosphat(en) und (c) Cetylalkohol als Bestandteil (a) gehärtete Palmölglyceride (INCI: Hydrogenated Palm Glycerides) (Mono-, Di- und Triglyceride von Fettsäuren, die in Palmöl enthalten sind, also z.B. die Mono-, Di- und Triglyceride der Ölsäure, der Palmitinsäure, der Stearinsäure, der Myristinsäure, der Lignocerinsäure und der Palmitoleinsäure). Gehärtete Palmölglyceride sind kommerziell verfügbar, z.B. unter dem Namen Mono-muls® 60-35C.

Ein erfindungsgemäßer Emulgator lässt sich bereits bei geringer Einsatzdosierung zur Herstellung von stabilen, hautglättenden O/W-Emulsionen verwenden.

Der erfindungsgemäße O/W-Emulgator enthält vorzugsweise kein Polyethylenglykol (PEG) und/oder kein sonstiges Glykol und/oder kein Paraffin und/oder kein Isoparaffin, besonders bevorzugt keine dieser Substanzen.

Vorzugsweise umfasst oder besteht ein erfindungsgemäßer O/W-Emulgator aus:

(a) 35 - 45 Gew.-% gehärteten Palmölglyceriden,
(b) 15 - 35 Gew.-% Kalium-Cetylphosphaten,
(c) 25 - 30 Gew.-% Cetylalkohol,
(d) 5 - 10 Gew.-% Kaliumphosphaten,
(e) 0 - 15 Gew.-% sonstigen Stoffen,

wobei sich die Gewichtsprozentangaben auf die Gesamtmasse des O/W-Emulgators beziehen.

Bestandteil (e) umfasst vorzugsweise Wasser, bevorzugt ist ein Wassergehalt im Bereich von 0,25 bis 10 Gew.-%, besonders bevorzugt im Bereich von 0,50 bis 5,0 Gew.-%, jeweils bezogen auf die Gesamtmasse des O/W-Emulgators, d.h. Gesamtmasse der Bestandteile (a) bis (e).

Ein erfindungsgemäßer O/W-Emulgator enthält als Bestandteil (b) vorzugsweise Kalium-Monocetylphosphat und Kalium-Dicetylphosphat, vorzugsweise in einem Gewichtsverhältnis des Kalium-Monocetylphosphats zum Kalium-Dicetylphosphat von 2,5 : 1 oder größer, bevorzugt in einem Gewichtsverhältnis im Bereich von 10 : 1 bis 2,5 : 1, weiter bevorzugt im Bereich von 6 : 1 bis 3 : 1, insbesondere bevorzugt im Bereich von 5 : 1 bis 3,5 : 1.

Ein erfindungsgemäßer O/W-Emulgator enthält in bzw. als Bestandteil (d) vorzugsweise Kaliumhydrogenphosphat, wobei der Anteil an Kaliumhydrogenphosphaten (Summe aus Kaliummonohydrogenphosphat und Kaliumdihydrogenphosphat) bevorzugt bei zumindest 3 Gew.-% liegt, bevorzugt im Bereich von 4 bis 15 Gew.-%, besonders bevorzugt im Bereich von 5 bis 10 Gew.-%, jeweils bezogen auf die Gesamtmasse des O/W-Emulgators.

Ein erfindungsgemäßer O/W-Emulgator wird vorzugsweise durch Vermischen der jeweiligen Bestandteile (a) bis (d) sowie gegebenenfalls (e) hergestellt . Alternativ wird zur Herstellung eines erfindungsgemäßen Emulgators vorzugsweise zunächst Phosphorsäure mit einer überschüssigen Menge an Cetylalkohol umgesetzt, wobei primär ein Reaktionsgemisch entsteht, welches Phosphorsäuremonohexadecylester, eine nicht umgesetzte Menge an Cetylalkohol und bevorzugt einen Anteil an Phosphorsäuredihexadecylester umfasst, wobei das Gewichtsverhältnis von Phosphorsäuremonohexadecylester zu Phosphorsäuredihexadecylester vorzugsweise zumindest 2,5 : 1 beträgt, siehe dazu oben. Dieses Reaktionsgemisch wird anschließend vorzugsweise mit Kaliumhydroxid (bevorzugt in Form von Kalilauge) oder Kaliumcarbonat versetzt, so dass ein erfindungsgemäßer Emulgator entsteht.

Der erfindungsgemäße O/W-Emulgator weist die folgenden verbesserten Eigenschaften im Vergleich zu dem O/W-Emulgator gemäß WO 2004/075868 auf:

• niedrigere Viskosität einer entsprechenden Emulsion bei gleicher Einsatzkonzentration des Emulgators;
• höhere Langzeitstabilität der entsprechenden Emulsionen, insbesondere von Emulsionen enthaltend wasserlösliche UV-Filter, speziell sulfonierte UV-Filter;
• verbesserte Wärmestabilität der entsprechenden Emulsionen, insbesondere bei Temperaturen oberhalb von 40°C:

deutlich geringere Schlierenbildung, keine oder nur sehr schwache Neigung zur Phasenseparation; bessere Schaukeltest-Stabilität;

• verstärkte Ausbildung liquidkristalliner Strukturen.

Ein höherer Anteil an liquidkristallinen Strukturen in O/W-Emulsionen trägt zu einer hohen Stabilität der Emulsionen bei; ferner bewirkt ein höherer Anteil an liquidkristallinen Strukturen ein besseres Feuchthaltvermögen der Haut (Moisturizing).

Zur Beurteilung der Langzeitstabilitat wird die Wärmestabilität der jeweiligen Emulsionen enthaltend den jeweils zu untersuchenden Emulgator bei konstanter Lagerungstemperatur über einen Lagerungszeitraum von zumindest 2 Monaten und bis zu 6 Monaten bei konstanter Temperatur, jeweils bei 5°C, 20°C, 40°C, 45°C, 50°C, jeweils im Dunkeln und zusätzlich bei Raumtemperatur (ca. 20°C) im Tageslicht beobachtet. Die Bewertung der Stabilität erfolgte regelmäßig nach 2 Wochen, 1 Monat, 2 Monaten und 3 Monaten sowie gegebenenfalls nach 6 Monaten.

Zur Beurteilung der Schaukeltest-Stabilität wird die jeweilige Emulsion zunächst für 24h bei -20°C, und anschließend für 24h bei +40°C gelagert (dies entspricht einem Zyklus), dann wieder 24h bei -20°C und so weiter. Insgesamt werden bis zu 10 Zyklen durchgeführt und dabei die Anzahl der Zyklen notiert, bis eine Separation der jeweiligen Emulsion beobachtet wird, d.h. ein Zusammenbruch der Emulsion durch Phasentrennung erfolgt. Wenn eine Emulsion mit "10 Zyklen" bewertet wird zeigt sie eine sehr gute Stabilität im Schaukeltest.

Der erfindungsgemäße O/W-Emulgator weist zusätzlich zu den vorstehend genannten Vorteilen die folgenden verbesserten Eigenschaften im Vergleich zu reinem Kaliummonocetylphosphat auf:

- bessere Öllöslichkeit bei niedrigeren Temperaturen;
- bewirkt in entsprechenden Emulsionen ein besseres / angenehmeres Hautgefühl;
- in Emulsionen mit Pigmenten (wie z.B: ZnO, $TiO_2$): leichtere Herstellbarkeit der Emulsion (geringere Scherkräfte erforderlich bei der Herstellung der Emulsion), höhere Stabilität von Emulsion enthaltend Pigmente;
- signifikant stärkere Ausbildung liquidkristalliner Strukturen;
- bei Kombination mit Stearinsäure: deutlich geringere Neigung zur Partikelbildung;
- bei Kombination mit Stearinsäure: deutlich geringere Neigung zur Verfärbung im Tageslicht.

Die Einsetzbarkeit erfindungsgemäßer O/W-Emulgatoren ist vielseitig, insbesondere sind sie für höherviskose Cremes, mittelviskose Milchen und Lotionen sowie dünnflüssige, sprühfähige Lotionen einsetzbar.

Der Einsatz der erfindungsgemäßen O/W-Emulgatoren ermöglicht eine gute Dispergierung von Feststoffen in den resultierenden Emulsionssystemen.

Bei Einsatz der erfindungsgemäßen O/W-Emulgatoren wird regelmäßig die Verteilbarkeit eingearbeiteter Wirkstoffe erhöht.

Bei Einsatz der erfindungsgemäßen O/W-Emulgatoren können Formulierungen (Emulsionen) unter Verwendung von polaren und unpolaren Ölen hergestellt werden.

Die erfindungsgemäßen O/W-Emulgatoren sind mit Hydrogelbildnern und Hydrokolloiden kompatibel.

Besonders vorteilhaft ist eine Kombination der erfindungsgemäßen O/W-Emulgatoren mit UV-Filtern (UV/A-, UV/B- und Breitbandfiltern) (Lichtschutzmitteln). Der Einsatz der erfindungsgemäßen Emulgatoren führt dabei regelmäßig zu einer Verbesserung der Wasserfestigkeit entsprechender Sonnenschutzprodukte.

Der erfindungsgemäße Emulgator wird vorzugsweise zusammen mit Lichtschutzmitteln formuliert. Bevorzugte Lichtschutzmittel sind z.B. organische UV-Absorber aus der Klasse der 4-Aminobenzoesäure und Derivate, Salicylsäure-Derivate, Benzophenon-Derivate, Dibenzoylmethan-Derivate, Diphenylacrylate, 3-lmidazol-4-yl-acrylsäure und deren Ester, Benzofuran-Derivate, Benzylidenmalonat-Derivate, polymere UV-Absorber (enthaltend einen oder mehrere Silizium-organische Reste), Zimtsäure-Derivate, Campher-Derivate, Trianilino-s-Triazin-Derivate, 2-Hydroxyphenylbenzotriazol-Derivate, 2-Phenylbenzimidazol-5-sulfonsäure und deren Salze, Anthranilsäurementhylester, Benzotriazolderivate.

Besonders geeignete UV-Filter sind dabei

- p-Aminobenzoesäure
- 3-(4'-Trimethylammonium)-benzyliden-bornan-2-on-methylsulfat
- Salicylsäure-homomenthylester (Neo Heliopan®HMS)
- 2-Hydroxy-4-methoxy-benzophenon (Neo Heliopan®BB)
- 2-Phenylbenzimidazolsulfonsäure (Neo Heliopan®Hydro)
- Terephthalyliden-dibornansulfonsäure und Salze (Mexoryl®SX)
- 4-tert.-Butyl-4'-methoxydibenzoylmethan (Neo Heliopan®357)
- 3-(4'-Sulfo)benzyliden-bornan-2-on und Salze
- 2-Ethylhexyl-2-cyano-3,3-diphenylacrylat (Neo Heliopan®303)
- N-[(2 und 4)-[2-(oxoborn-3-yliden)methyl]benzyl]-acrylamid-Polymer
- p-Methoxyzimtsäure-2-ethylhexylester (Neo Heliopan®AV)
- p-Aminobenzoesäure-ethylester (25 Mol) ethoxyliert
- p-Methoxyzimtsäure-isoamylester (Neo Heliopan®E1000)
- 2,4,6-Trianilino-(p-carbo-2'-ethylhexyl-1'-oxy)-1,3,5-triazin (Uvinul®T150)
- Phenol, 2-(2H-benzotriazol-2-yl)-4-methyl-6-(2-methyl-3(1,3,3,3-tetramethyl-1- (trimethylsilyl)-oxy)-disiloxanyl)-propyl), (Mexoryl®XL)

- 4,4'-[(6-[4-(1,1-Dimethyl)-aminocarbonyl)-phenylamino]-1,3,5-triazin-2,4-diyl)-diimino]-bis-(benzoesäure-2-ethylhexylester), (UvasorbHEB)
- 3-(4'-Methylbenzyliden)-d,1-campher (Neo Helipan®MBC)
- 3-Benzylidencampher
- Salicylsäure-2-ethylhexylester (Neo Helipan®OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol), (Tinosorb®M)
- Phenylen-bis-benzimidazyl-tetrasulfonsäure-dinatriumsalz (Neo Heliopan® AP)
- 2,4-Bis-[{(4-(2-Ethyt-hexy)oxy)-2-hydroxy}-phenyl]-6-(4-methoxyphenyl)-1,3,5-triazin, (Tinosorb®S)
- Benzylidenmalonat-Polysiloxan (Parsol®SLX)
- Menthylanthranilat (Neo Heliopan®MA)
- 2-(4-Diethylamino-2-hydroxybenzoyl)-benzoesäure-hexylester (Uvinul® A Plus)
- Indanylidenverbindungen gemäß DE 100 55 940 (= WO 02/38537)

Ein besonderer Vorteil von Emulsionen enthaltend erfindungsgemäße Emulgatoren und wasserlösliche UV-Filter, insbesondere sulfonierte wasserlösliche UV-Filter, ist deren hohe Langzeitstabilität, was sich insbesondere bemerkbar macht durch eine verbesserte Wärmestabilität vor allem bei Temperaturen oberhalb von 40°C; es wird nur eine geringe Neigung zur Schlierenbildung, keine oder eine nur sehr schwache Neigung zur Phasenseparation und eine signifikant bessere Schaukeltest-Stabilität beobachtet im Vergleich zu entsprechenden Emulsionen welche einen O/W-Emulgator gemäß WO 2004/075868 enthalten. Die genannten Vorteile betreffen insbesondere erfindungsgemäße Emulsionen, siehe dazu unten.

Die Gesamtmenge aller wasserlöslichen UV-Filtersubstanzen in einer bevorzugten (erfindungsgemäßen) Emulsion liegt vorzugsweise im Bereich von 0,1 bis 12,0 Gew.-%, bevorzugt 0,5 bis 8,0 Gew.-% , bezogen auf das Gesamtgewicht der (erfindungsgemäßen) Emulsion. Dabei bevorzugt sind (einfach oder mehrfach) sulfonierte wasserlösliche UV-Filter, welche bevorzugt gewählt sind der Gruppe bestehend aus Phenylen-bis-benzimidazyl-tetrasulfonsäure-dinatriumsalz bzw. deren Salzen und/oder die entsprechende Disulfonsäure bzw. deren Salzen und/oder 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)-benzolsulfonsäure bzw. deren Salzen und/oder 2-Methyl-5-(2-oxo-3-bornyliden-methyl)-benzolsulfonsäure bzw. deren Salzen und/oder Benzol-1,4-di-(2-oxo-3-bornylidenmethyl)-10-sulfonsäure bzw. deren Salzen.

Der erfindungsgemäße Emulgator kann ferner in kosmetische und/oder dermatologische Zubereitungen eingearbeitet werden, die Pigmente, bevorzugt feinteilige Pigmente enthalten. Hierbei kann es sich um organische oder anorganische Pigmente handeln. Bevorzugtes organisches Pigment ist 2,2'-Methylen-bis-[6-(2H-benzotriazol-2-yl)-4-(1,1,3,3tetramethylbutyl)-phenol] (Tinosorb® M). Geeignete anorganische Pigmente oder Mikropigmente auf Basis von Metalloxiden und/oder anderen in Wasser schwerlöslichen oder unlöslichen Metallverbindungen sind insbesondere Oxide des Titans ($TiO_2$), Zinks (ZnO), Eisens (z.B. $Fe_2O_3$), Zirkoniums ($ZrO_2$) Siliciums ($SiO_2$) Mangans (z.B. MnO), Aluminiums ($Al_2O_3$), Cers (z.B. $Ce_2O_3$), Mischoxiden der entsprechenden Metalle sowie Abmischungen aus solchen Oxiden. Diese Pigmente sind röntgenamorph oder nichtröntgenamorph. Besonders bevorzugt sind feinteilige Pigmente auf der Basis von $TiO_2$ und ZnO.

Der erfindungsgemäße O/W-Emulgator kann ferner in kosmetische und/oder dermatologische Zubereitungen (Emulsionen) eingearbeitet werden, die wie üblich zusammengesetzt sind und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können derartige Zubereitungen, je nach ihrem Aufbau beispielsweise als Hautschutzcreme, Reinigungsmilch, Sonnenschutzlotion, Nährcreme, Tages- oder Nachtcreme usw. verwendet werden. So können derartige Zubereitungen (Emulsionen) beispielsweise als Lotion, Milch, Creme, Hydrodispersionsgel, Balm, Spray, Schaum, Haar-Shampoo, Haar-Pflegemittel, Haar-Conditioner, Roll-on, Stick oder Make-up vorliegen.

Besonders vorteilhaft sind kaufmännische und logistische Vorteile, da lediglich ein Emulgator beschafft und bevorratet werden muss, um Lotionen und Cremes herzustellen.

Der erfindungsgemäße O/W-Emulgator besitzt eine sehr gute Hautverträglichkeit.

Ein weiterer Vorteil des erfindungsgemäßen O/W-Emulgatores ist, dass dieser in verschiedenen Formen konfektioniert werden kann, bevorzugt sind Pulver, Pastille, Extrudat, Schuppe (Flake). Die Pastillenform ist auch die bevorzugte Form der Konfektionierung des erfindungsgemäßen O/W-Emulgators.

Aufgrund der chemischen Zusammensetzung der erfindungsgemäßen O/W-Emulgatoren sind ihre physikalischen und chemischen Eigenschaften weitgehend bestimmt, gewisse Variationen sind aber nichtsdestoweniger möglich.

Als besonders vorteilhaft haben sich erfindungsgemäße O/W-Emulgatoren erwiesen, die eine, mehrere oder vorzugsweise sämtliche der folgenden Bedingungen erfüllen:

- nach Lösen in neutralisiertem Wasser bei einer Konzentration von 10 Gew.-%, bezogen auf die fertige Lösung, liegt der pH-Wert im Bereich von 5,0 - 6,5;

- die Verseifungszahl bei einer Verseifungsdauer von 1 Stunde liegt im Bereich von 125,0 bis 155,0 (Testmethode 211);

- die Säurezahl bei Verwendung des Lösemittelsystems Diethylether:Ethanol:Wasser 1:1:1 (v/v/v) liegt im Bereich von 50,0 bis 80,0 mg KOH/g (Testmethode 228).

Hinsichtlich der angegebenen Testmethoden 211 bzw. 228 verweisen wir auf die Beispiele weiter unten.

Der Schmelzpunkt eines erfindungsgemäßen O/W-Emulgators ist vorzugsweise so eingestellt, dass er im Bereich von 75 bis 80°C liegt. Die Einstellung des Schmelzpunkts wird dabei insbesondere durch den Gehalt an Cetylalkohol im O/W-Emulgator gesteuert.

Die Einarbeitung eines erfindungsgemäßen O/W-Emulgators in eine O/W-Emulsion kann wahlweise über die Wasserphase oder die Ölphase erfolgen.

Die der Erfindung zugrunde liegende gestellte Aufgabe wird gemäß einem zweiten Aspekt durch eine O/W-Emulsion gelöst, umfassend:

- eine Wasserphase
- eine in der Wasserphase dispergierte Ölphase und
- 0,25-15 Gew.-%, vorzugsweise 1-15 Gew.-% eines erfindungsgemäßen O/W-Emulgators, wobei die Gewichtsprozentangabe auf die Gesamtmasse der O/W-Emulsion bezogen ist.

Zum Stabilisieren von Emulsionen genügt dabei häufig ein Anteil von 0,25 bis 0,50 Gew.-% des erfindungsgemäßen O/W-Emulgators.

Zur Herstellung erfindungemäßen O/W-Emulsionen werden typischerweise Einsatzkonzentrationen des erfindungsgemäßen O/W-Emulgators angewandt, die im Bereich von 0,5 bis 3,0 Gew.-% liegen.

Für mittelviskose Lotionen (als Beispiel erfindungsgemäßer-Emulsionen) werden dabei vorzugsweise 0,5 bis 4,0 Gew.-%, vorzugsweise zwischen 1,0 und 2,0 Gew.-%, des erfindungsgemäßen O/W-Emulgators eingesetzt.

Salben und Cremes enthalten (als weitere Beispiele erfindungsgemäßer Emulsionen) regelmäßig 1,0 bis 15,0 Gew.-%, vorzugsweise 1,0 bis 3,0 Gew.-% des erfindungsgemäßen O/W-Emulgators.

Es kann in manchen Ausführungsformen vorteilhaft sein, den erfindungsgemäßen O/W-Emulgator in der Wasserphase zu verarbeiten und dabei gleichzeitig einen Alkohol, vorzugsweise ein Diol oder Polyol, mit zu verarbeiten. Auf diese Weise kann die Lösungstemperatur des erfindungsgemäßen O/W-Emulgators in der Wasserphase herabgesetzt werden. Der erfindungsgemäße O/W-Emulgator wird in diesem Fall vorzugsweise in einem Alkohol, vorzugsweise einem Diol oder Polyol, bevorzugt Glycerin und/oder 1,2-Propylenglycol und/oder Pentylenglycol (1,2-Pentandiol) vorgelöst. Die flüssige Phase aus O/W-Emulgator und Alkohol wird dann vorzugsweise anschließend mit den sonstigen Bestandteilen der Wasserphase vermischt. Alternativ können der oder die Alkohol(e) auch vorab in die Wasserphase eingearbeitet werden, wobei der erfindungsgemäße O/W-Emulgator dann zu der resultierenden Wasserphase hinzugegeben wird.

Die erfindungsgemäßen O/W-Emulsionen zeichnen sich unter anderem aus durch:

- eine gute Viskositätsstabilität,
- eine hohe pH-Stabilität bzw. pH-unabhängige Stabilität,
- eine gute Temperaturstabilität,
- eine sehr feine und homogene Emulsionsstruktur mit glänzender Oberfläche, sowie
- durch kaufmännische und logistische Vorteile, da lediglich ein Emulgator beschafft und bevorratet werden muss, um Lotionen und Cremes herzustellen.

Neben der Wasserphase, der in der Wasserphase dispergierten Ölphase und dem O/W-Emulgator umfassen bevorzugte O/W-Emulsionen zusätzlich 0,1 - 10 Gew.-% eines Stabilisierungsmittels und/oder 1 - 10 Gew.-% eines Co-Emulgators, wobei die Gewichtsprozentangabe auf die Gesamtmasse der O/W-Emulsion bezogen ist.

Der pH-Wert einer erfindungsgemäßen O/W-Emulsion kann in weiten Bereichen variieren. Vorteilhafterweise ist der pH auf einen Wert zwischen 3 und 11, vorzugsweise zwischen 4 und 9, weiter vorzugsweise zwischen 4 und 7 eingestellt.

Als Co-Emulgatoren können z.B. Glycerinmonostearate oder andere Glycerinmonoester von Fettsäuren, Stearinsäure oder andere Fettsäuren (unverseift oder partialverseift), Wachse oder Fettalkohole zum Einsatz kommen.

Als Stabilisierungsmittel können Hydrogelbildner wie z.B. Carbomere, Acrylat-Crosspolymere, Xantane, Alginate usw. verwendet werden.

Bevorzugte O/W-Emulsionen sind frei von Polyethylenglycol (PEG), sonstigen Glycolen, Paraffin und/oder Isoparaffin. Vorzugsweise ist keine der genannten Substanzen anwesend.

Die erfindungsgemäße O/W-Emulsion kann weitere Bestandteile umfassen, insbesondere:

- dispergierte Feststoffe
  und/oder
- UV-A- und/oder UV-B-Filter
  und/oder
- ein Antioxidationsmittel,
  und/oder
- ein Parfümöl,
  und/oder
- sonstige Hilfsstoffe.

Im Falle der Kombination der erfindungsgemäßen Emulgatoren in den erfindungsgemäßen Emulsionen mit Lichtschutzfiltern (UV/A- und/oder UV/B-Filter) wird deren Verteilbarkeit auf der Haut verbessert und eine erhöhte Wasserfestigkeit im Vergleich zu sonstigen Emulsionen erreicht.

Der erfindungsgemäße Emulgator hat in einer erfindungsgemäßen Emulsion allein oder gemeinsam mit anderen kosmetischen Hilfsstoffen ferner die folgende Wirkung:

Steigerung des Sonnenschutzfaktors von UV-Filtern (UVA- und/oder UVB-Schutz); Stabilisierung von UV-Filtern (verbesserte Photostabilisation); Verbesserung der Löslichkeit und/oder Suspension von festen UV-Filtern; Erhöhung der Wasserfestigkeit von Sonnenschutzprodukten; Unterstützung bei der Bildung einer Gelnetzwerkstruktur; Steigerung der Wirksamkeit von aktiven Stoffen, wie z.B. Antioxidantien, Konservierungsmittel, Aufheller (Skin Lightener) und Bräunungsmittel, Parfümöle, Chelatbildner; Steigerung der Substantivität von aktiven Wirkstoffen auf der Haut und/oder dem Haar; Verbesserung der Verteilung von kosmetischen Ölen (Pflanzenöle, Mineralöle, Emollients), Wirkstoffen, Vitaminen, Parfümölen und ätherischen Ölen auf der Haut; Unterstützung einer gleichmäßigen Verteilung von RepellentWirkstoffen; Beitrag zu einer optimalen Verteilung von Konservierungssystemen in der Wasserphase; Unterstützung der Barriere-Funktion der Haut; Reduktion der Agglomerationsrate von anorganischen UV-Filtern (Titandioxid, Zinkoxid) und Farbpigmenten; Unterstützung der Verteilung von Aluminiumsalzen in Antiperspirant-Produkten; Verträglichkeit mit Alkoholen, auch mit Ethanol; verbesserte Stabilisierung von Emulsionen als Haupt- oder CoEmulgator.

Die erfindungsgemäßen Emulsionen sind vom Typ "Öl-in-Wasser" (O/W), oder vom Typ Wasser-in-Öl-in-Wasser (W/O/W). Die erfindungsgemäßen Emulsionen können insbesondere formuliert sein als, Stift, Stick, Aerosol, Spray, Schaum, Tränkungslösung, z.B. für kosmetische Tücher, Reinigungsmittel wie z.B. Seife, Syndets, Hautpflegemittel, Creme, Lotion, Milch, Emulsionsschaum, Mikroemulsion, Paste, Gel (z.B. Hydro- oder Hydrodispersionsgel), Balsam, Serum, Roll-On, Pumpspray, Aerosol (schäumend, nicht schäumend oder nach-schäumend), Hautpflegemittel, Fußpflegemittel (inklusive Keratolytika, Desodorantien), Insekten abwehrendes Mittel, Sonnenschutzmittel, Aftersun-Präparat, Rasiermittel, Haarentfernungsmittel, Haarpflegemittel wie z.B. Shampoo, 2-in-1 Shampoo, Anti-Schuppen-Shampoo, Babyshampoo, Shampoo für trockene Kopfhaut, Shampookonzentrat, Conditioner, Haarkur, Haarspülung, Frisiercreme, Haarglättungsmittel (Entkräuselungsmittel, Relaxer), Haarfestiger (Spray), Styling-Aid (z.B. Gel), als Blondiermittel, Haaraufheller, Haarconditioner, Haarmousse, Haartönung, Deodorant und / oder Antitranspirant, Aftershave Balm, Pre- und Aftershave Lotion, Augenpflege, Make-Up, Make-Up Entferner, Babyartikel, Badeartikel, oder Maske.

Bevorzugte erfindungsgemäßen Emulsionen liegen in einer der folgenden Formen vor: Sonnenschutzmilch, Sprühfähige Sonnenmilch, Sonnenschutzlotion, Sonnenschutzcreme, Gesichtscreme mit Sonnenschutz, Haargel, Haarcreme, Haarkurspülung, Deostift, Deo Roll-on, Sprühbare Deo-Lotion, Pflegelotion für Feuchttücher, Body-Lotion, Hautpflegecreme, Getönte Tagescreme, Mascara.

Es ist gegebenenfalls möglich und vorteilhaft, derartige Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkprodukts vorliegen.

Zur Anwendung werden die beispielhaft erwähnten kosmetischen und dermatologischen Zubereitungen in der für Kosmetika üblichen Weise auf die Haut und/oder die Haare in ausreichender Menge aufgebracht.

Die Lipidphase kann vorteilhaft gewählt werden aus folgender Substanzgruppe:

- Mineralöle, Mineralwachse;
- Öle, wie Triglyceride der Caprin- oder der Caprylsäure, ferner natürlicher Öle wie z.B. Rizinusöl;
- Fette, Wachse und andere natürliche und synthetische Fettkörper, vorzugsweise Ester von Fettsäuren mit Alkoholen niedriger C-Zahl (<10), z.B. mit Isopropanol, Propylenglykol oder Glycerin, oder Ester von Fettalkoholen mit Alkansäuren niedriger C-Zahl (<10) oder mit Fettsäuren;
- Alkylbenzoate;

- Silikonöle wie Dimethylpolysiloxan, Diethylpolysiloxan, Diphenylpolysiloxan sowie Mischformen daraus.

Die Lipidphase der Emulsionen, Oleogele bzw. Hydrodispersionen oder Lipodispersionen im Sinne der vorliegenden Veröffentlichung werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, -palmitat, - stearat, -oleat, n-Butylstearat, n-Hexyllaurat, n-Decyloleat, Isooctylstearat, Isononylstearat, Isononylisononanat, 2-Ethyl-hexylpalmitat, Ethylhexyllaurat, 2-Hexyldecylstearat, 2-Octyldodecylpalmitat, Oleyloleat, Oleylerucat, Erucyloleat sowie synthetische, halbsynthetische und natürliche Gemische solcher Ester, z.B. Jojobaöl, 2-Ethylhexyl-2-Ethylhexanoat (z.B. Dragoxat EH), Ethylhexyl Isononanoat (z.B: Dragoxat 89), Cetearyl-2-Ethylhexanoat, Düsopropyladipat, Triisonananoin.

Ferner kann die Lipidphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einsetzbar.

Vorteilhaft wird Cyclomethicon (Octamethylcyclotetrasiloxan) als zu verwendendes Silikonöl eingesetzt. Aber auch andere Silikonöle sind ähnlich vorteilhaft zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase von Zubereitungen im Sinne dieser Veröffentlichung enthält gegebenenfalls vorteilhaft wasserlösliche Pflanzenextrakte, Alkohole, Diole oder Polyole (Niederalkyl), sowie deren Ether, vorzugsweise Ethanol, Isopropanol, Propylenglykol, Glycerin, Ethylenglykol-monoethyl- oder - monobutylether, Propylenglykolmonomethyl, -monoethyl- oder - monobutylether, Diethylenglykolmonomethyl- oder -monoethylether und analoge Produkte, ferner Alkohole (Niederalkyl), z.B. Ethanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxyropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen und dermatologischen Zubereitungen (Emulsionen) im Sinne dieses Textes können kosmetische Hilfsstoffe enthalten, wie sie üblicherweise in solchen Zubereitungen verwendet werden, z.B. Konservierungsmittel, Antioxidantien, Vitamine, Bakterizide, Parfüme, Substanzen zum Verhindern des Schäumens, Farbstoffe, Pigmente, die eine färbende Wirkung haben, Verdickungsmittel, Tenside, Emollients, Emulgatoren, anfeuchtende und/oder feuchthaltende Substanzen, Feuchtigkeitsvermittler, Fette, Öle, Wachse, Pflanzenextrakte oder andere übliche Bestandteile einer kosmetischen oder dermatologischen Formulierung wie Alkohole, Niederalklyalkohole, Polyole, Niederalkylpolyole, Polymere, Schaumstabilisatoren, Komplexbildner, Elektrolyte, organische Lösungsmittel, Treibgase, Silikone oder Silikonderivate.

Die jeweils einzusetzenden Mengen an kosmetischen oder dermatologischen Hilfs- und Trägerstoffen und Parfüm können in Abhängigkeit von der Art des jeweiligen Produktes vom Fachmann leicht ermittelt werden.

Ein zusätzlicher Gehalt an Antioxidantien ist im allgemeinen bevorzugt. Als günstige Antioxidantien können alle für kosmetische und/oder dermatologische Anwendungen geeignete oder gebräuchliche Antioxidantien verwendet werden.

Die Menge der Antioxidantien (eine oder mehrere Verbindungen) in den Zubereitungen beträgt vorzugsweise 0,001 - 30 Gew.-%, besonders bevorzugt 0,05 - 20 Gew.-%, insbesondere 1-10 Gew.-%, bezogen auf das Gesamtgewicht der Zubereitung.

Vorteilhaft werden die Antioxidantien aus der folgenden Gruppe gewählt: Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Carnosin, L-Carnosin, Anserin) und deren Derivate, Carotinoide, Carotine (z.B. alpha-Carotin, beta-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4Dihydroxyphenylessigsäure-, Ferulasäure-, Sinapinsäure, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), Catecholoxime oder Catecholoximether (z.B. 3,4-Dihydroxybenzaldoxim oder 3,4-Dihydroxybenzaldehyd-O-ethyloxim), 2-Hydrazino- 1,3thiazol und Derivate, ferner (Metall-)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z.B. Ascorbylpalmitat, Magnesiumascorbylphosphat, Ascorbylacetat), Tocopherole und Derivate (z.B. Vitamin-E-Acetat), Vitamin A und Derivate (z.B. Vitamin-A-Palmitat), Rutinsäure und deren Derivate, Flavonoide (z.B. Quercetin, alpha-Glucosylrutin) und deren Derivate, Phenolsäuren (z.B. Gallussäure, Ferulasäure) und deren Derivate (z.B. Gallussäurepropylester, -ethylester, -octylester), Furfurylidenglucitol, Dibutylhydroxytoluol, Butylhydroxyanisol, Harnsäure und deren Derivate, Mannose und deren Derivate, Zink und dessen Derivate (z.B. ZnO, $ZnSO_4$), Selen und dessen Derivate (z.B. Selenomethionin), Stilbene und deren Derivate (z.B. Stilbenoxid, Resveratrol).

Ebenfalls vorteilhafte Antioxidantien sind beschrieben in EP 0 900 781, EP 1 029 849, EP 1 066 821, WO 01/43712, WO 01/70176, WO 01/98235 oder auch in WO 01/98258.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Sofern Vitamin A bzw. Vitamin-A-Derivate, oder Carotine bzw. deren Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Weiter bevorzugte Ausgestaltungen der Erfindung ergeben sich aus den beigefügten Patentansprüchen.

Die Erfindung wird nachfolgend anhand von Beispielen näher erläutert. Sofern nicht anders angegeben, beziehen sich alle Angaben auf das Gewicht.

Beispiel 1: Erfindungsgemäße Emulgatoren

| Bestandteil | Beispiel 1A (Gew.-%) | Beispiel 1B (Gew.-%) |
|---|---|---|
| Gehärtete Palmölglyceride | 39,8 | 40,0 |
| Kalium-Monocetylphosphat | 23,5 | 14,8 |
| Kalium-Dicetylphosphat | 4,5 | 3,6 |
| Cetylalkohol | 25,5 | 29,5 |
| Kaliumphosphate | 5,5 | 8,9 |
| Wasser | 0,55 | 0,7 |
| Sonstige | Ad 100 | Ad 100 |

Beispiel 2: Vergleichsemulgator gemäß WO 2004/075868 (nicht erfindungsgemäß)

| Bestandteil | Gew.-% |
|---|---|
| Gehärtete Palmölglyceride | 40,0 |
| Kalium-Monocetylphosphat | 40,0 |
| Cetylalkohol | 15,0 |
| Wasser | 5,0 |

Beispiel 3: Stabilitätstest der Emulsionen I - IV im Vergleich

| | Rohstoff | INCI | I | II | III | IV |
|---|---|---|---|---|---|---|
| A | Emulgator gemäß Beispiel 1 A | | 1,0 | | | |
| | Emulgator gemäß Beispiel 1 B | | | 0,5 | | |
| | Emulgator gemäß Beispiel 2; WO 2004/075868 | Potassium Cetyl Phosphate, Hydrogenated Palm Glyceride | | | 1,0 | 0,5 |
| | Finsolv TN | C12-15 Alkyl Benzoate | 5,0 | 5,0 | 5,0 | 5,0 |
| | Copherol 1250 | Tocopheryl Acetate | 0,5 | 0,5 | 0,5 | 0,5 |
| | Lanette O | Cetearyl Alcohol | 1,0 | 1,0 | 1,0 | 1,0 |
| | Neutralöl | Caprylic/Capric Triglyceride | 2,0 | 2,0 | 2,0 | 2,0 |
| | Dow Corning 246 Fluid | Cyclohexasiloxane | 2,0 | 2,0 | 2,0 | 2,0 |
| | Neo Heliopan® 357 | Butyl Methoxydibenzoylmethane | 1,0 | 1,0 | 1,0 | 1,0 |
| | Neo Heliopan® OS | Ethylhexyl Salicylate | 5,0 | 5,0 | 5,0 | 5,0 |
| | Neo Heliopan® AV | Ethylhexyl Methoxycinnamate | 3,0 | 3,0 | 3,0 | 3,0 |
| | Neo Heliopan® MBC | 4-Methylbenzoylidene Camphor | 1,5 | 1,5 | 1,5 | 1,5 |
| | EDTA BD | Disodium EDTA | 0,1 | 0,1 | 0,1 | 0,1 |
| | Keltrol T | Xanthan Gum | 0,2 | 0,2 | 0,2 | 0,2 |
| | Carbopol EDT 2050 | Carbomer | 0,2 | 0,2 | 0,2 | 0,2 |
| | | | | | | |
| B | Wasser, demin. | Water (Aqua) | 59,9 | 60,4 | 59,9 | 60,4 |
| | Glycerin | Glycerin | 4,7 | 4,7 | 4,7 | 4,7 |
| | Dragocid Liquid | Phenoxyethanol Methylparaben, Ethylparaben Butylparaben, Propylparaben, Isobutylparaben | 0,7 | 0,7 | 0,7 | 0,7 |
| | Neo Heliopan® AP, 22%ige Lösung neutralisiert mit TEA | Disodium Phenyl Dibenzimidazole Tetrasulfonate | 4,55 | 4,55 | 4,55 | 4,55 |
| | Neo Heliopan® Hydro 30%ige Lösung neutralisiert mit TEA | Phenylbenzimidazole Sulfonic Acid | 6,67 | 6,67 | 6,67 | 6,67 |
| | | | | | | |
| C | Triethanolamin | Triethanolamine | 0,5 | 0,5 | 0,5 | 0,5 |
| | | | | | | |
| D | Parfümöl | Fragrance | 0,4 | 0,4 | 0,4 | 0,4 |
| | Alpha-Bisabolol nat. | Bisabolol | 0,1 | 0,1 | 0,1 | 0,1 |
| | | | | | | |
| | Angaben in Gew.-% | Total: | 100 | 100 | 100 | 100 |
| TEA: Triethanolamin | | | | | | |

Der pH-Wert der Emulsionen I - IV war über den Beobachtungszeitraum (3 Monate) stabil und lag bei 7,7 - 7,8.

Herstellungsvorschrift:

Phase A und B auf ca. 80°C erhitzen, Gelbildner (Xanthan Gum, Carbomer) in A eindispergieren, Phase B zu A geben und homogenisieren, dabei Teil C zugeben, Kaltrühren, dann bei 40-35 °C Teil D zugeben.

Bewertung der Langzeitstabilität:

| Temperatur | Dauer/Zeitraum | I | II | III | IV |
|---|---|---|---|---|---|
| 5 °C | 3 Monate | A | A | S1 | S2 |
| 40 °C | 2 Monate | S0 | S0 | S1 | S2 |
| 40 °C | 3 Monate | S0 | S0 | S2 | S3 |
| 50 °C | 1 Monat | A | A | S1 | S1 |
| 50 °C | 2 Monate | S0 | S0 | S3 | S3 |
| 50 °C | 3 Monate | S1 | S1 | S3 | P1 |

Bewertung:

A = gute Stabilität, keine nennenswerte Veränderung;

S0 = geringfügige Schlierenbildung, aber noch akzeptabel;

S1 = leichte Schlierenbildung;

S2 = deutliche Schlierenbildung;

S3 = starke Schlierenbildung;

P1 = Phasentrennung.

Schaukeltest (1 Zyklus = 24h bei -20°C, dann 24h bei +40°C):

| I | II | III | IV |
|---|---|---|---|
| 10 Zyklen | 10 Zyklen | 10 Zyklen | 8 Zyklen |

Die nicht erfindungsgemäßen Emulsionen III und insbesondere IV zeigten eine signifikant schlechtere Langzeitstabilität.

Beispiel 4: Stabilitätstest der Emulsionen V und VI im Vergleich

| | Rohstoff | INCI | V | VI |
|---|---|---|---|---|
| A | Emulgator gemäß Beispiel 1A | | 0,50 | - |
| | Emulgator gemäß Beispiel 2; WO 2004/075868 | | - | 0,50 |

(fortgesetzt)

| | Rohstoff | INCI | V | VI |
|---|---|---|---|---|
| | Dracorin GMS | Glyceryl Stearate | 0,50 | 0,50 |
| | Paraffinöl | Paraffinum Liquidum | 25,00 | 25,00 |
| | Abil 350 | Dimethicone | 2,00 | 2,00 |
| | Carbopol Ultrez 10 | Carbomer | 0,10 | 0,10 |
| | Keltrol RD | Xanthan Gum | 0,10 | 0,10 |
| | | | | |
| B | Demineralisiertes Wasseer | Water (Aqua) | 70,45 | 70,45 |
| | Dragocid Liquid | Phenoxyethanol, Methylparaben, Ethylparaben, Butylparaben, Propylparaben, Isobutylparaben | 0,80 | 0,80 |
| | | | | |
| C | NaOH, 10%ige wässrige Lösung | Sodium Hydroxide | 0,25 | 0,25 |
| | | | | |
| D | Parfüm | Fragrance (Parfum) | 0,30 | 0,30 |
| | | | | |
| | Summe | | 100,0 | 100,0 |
| | pH-Wert | | 6,2 | 6,3 |

Herstellungsvorschrift:

Phase A ohne Carbomer, Xanthan Gum einwiegen, erhitzen bis 80°C, dann Carbomer, Xanthan Gum zugeben und einhomognisieren. Phase B auf ca. 80°C erhitzen, B zu A geben und homogenisieren, dabei Teil C zugeben. Kaltrühren, bei 40-35 °C Teil D zugeben.

Der pH-Wert der Emulsionen V und VI war über den Beobachtungszeitraum (3 Monate) stabil.

Bewertung der Langzeitstabilität:

| Temperatur | Dauer / Zeitraum | V | VI |
|---|---|---|---|
| 40°C | 1 Monat | A | S1 |
| 40°C | 2 Monate | S0 | S2 |
| 50 E | 2 Wochen | A | V1 |
| 50°C | 1 Monat | S0 | S1 |
| 50°C | 2 Monate | S1 | S3 |
| 50°C | 3 Monate | S1 | P1 |

Bewertung:

A = gute Stabilität, keine nennenswerte Veränderung;

V1 = leichte Viskositätsabnahme;

S0 = geringfügige Schlierenbildung, aber noch akzeptabel;

S1 = leichte Schlierenbildung;

S2 = deutliche Schlierenbildung;

S3 = starke Schlierenbildung;

P1 = Phasentrennung.

Schaukeltest (1 Zyklus = 24h bei -20°C, dann 24h bei +40°C):

| V | VI |
|---|---|
| 10 Zyklen | 4 Zyklen |

Die nicht erfindungsgemäße Emulsion VI zeigte eine deutlich schlechtere Langzeitstabilität.

Formulierungsbeispiele:

Beispiel F1: Sonnenschutzmilch (O/W)
Beispiel F2: Sonnenschutzlotion (O/W)
Beispiel F3: Gesichtscreme (O/W) mit Sonnenschutz
Beispiel F4: Sonnenschutzmilch

| | Rohstoff | INCI Name | F1 | F2 | F3 | F4 |
|---|---|---|---|---|---|---|
| A | Abil 100 | Dimethicone | 0,3 | | 0,3 | |
| | Cetiol OE | Dicaprylyl Ether | 5,0 | 5,0 | 1,5 | |
| | Copherol 1250 | Tocopheryl Acetate | 0,5 | 0,5 | 0,5 | 0,5 |
| | Corapan TQ® | Diethylhexyl 2,6-naphthalate | 2,0 | 5,0 | 5,0 | 2,5 |
| | Cutina FS 45 | Palmitic Acid (and) Stearic Acid | | 2,0 | | |
| | Cutina MD | Glyceryl Stearate | 2,0 | 1,0 | 2,0 | |
| | Dragoxat 89 | Ethylhexyl Isononanoate | | | 1,5 | |
| | Edeta BD | Disodium EDTA | | | | 0,1 |
| | Emulgator gemäß Beispiel 1 A | | 1,5 | 0,3 | | |
| | Emulgator gemäß Beispiel 1B | | | | 1,5 | 0,5 |
| | Hostacerin DGMS | Polyglyceryl-2 Stearate | | | | 4,0 |
| | Keltrol T | Xanthan Gum | | | | 0,4 |
| | Lanette 16 | Cetyl Alcohol | 1,2 | | 1,5 | |
| | Lanette 0 | Cetearyl Alcohol | | 1,0 | | |
| | Miglyol 812 | Caprylic/Capric Triglyceride | | | | 5,0 |
| | Neo Heliopan® 357 | Butyl Methoxydibenzoylmethane | 1,0 | 0,8 | 2,0 | 1,5 |
| | Neo Heliopan® HMS | Homosalate | 7,0 | | 5,0 | 8,0 |
| | Neo Heliopan® MBC | 4-Methylbenzylidene Camphor | | 3,0 | | |
| | Neo Heliopan® OS | Ethylhexyl Salicylate | | | 5,0 | |

(fortgesetzt)

| | Rohstoff | INCI Name | F1 | F2 | F3 | F4 |
|---|---|---|---|---|---|---|
| | PCL Liquid | Cetearyl 2-Ethylhexanoate | 4,0 | 5,0 | | |
| | Prisorine 3505 | Isostearic Acid | | | | 0,5 |
| | SF 1214 | Cyclopentasiloxane (and) Dimethicone | | | | 1,0 |
| | Solbrol P | Propylparaben | 0,1 | 0,1 | 0,1 | 0,1 |
| | Tegosoft TN | C12-15 Alkyl Benzoate | | | | 4,0 |
| | Trilon BD | EDTA | | | 0,1 | |
| | Zinkoxid neutral | Zinc Oxide | | | | 7,0 |
| | | | | | | |
| B | 1,3 - Butylenglykol | Butylene Glycol | 3,0 | 3,0 | | |
| | Carbopol ETD 2050 | Carbomer | 0,2 | 0,2 | 0,3 | |
| | Glycerin | Glycerin | | | 3,0 | 4,0 |
| | Keltrol T | Xanthan Gum | 0,2 | 0,2 | 0,5 | |
| | Lanette E | Sodium Cetearyl Sulfate | | | | 0,75 |
| | Natronlauge, 10% aq. | Sodium Hydroxide | | | | 2,5 |
| | Neo Heliopan® AP | Disodium Phenyl Dibenzimidazole Tetrasulfonate | | | | 2,2 |
| | Neo Heliopan® AP, 10%ige Lösung neutralisiert mit NaOH | Disodium Phenyl Dibenzimidazole Tetrasulfonate | 22,0 | 22,0 | 25,0 | |
| | Phenoxyethanol | Phenoxyethanol | 0,7 | 0,7 | 0,7 | 0,7 |
| | Solbrol M | Methylparaben | 0,2 | 0,2 | 0,2 | 0,2 |
| | Wasser, dest. | Water (Aqua) | 45,9 | 47,2 | 40,4 | 54,05 |
| | | | | | | |
| C | Natronlauge, 10% | aq. Sodium Hydroxide | 2,8 | 2,4 | 3,5 | |
| | Parfumöl | Fragrance (Parfum) | | | | 0,5 |
| | | | | | | |
| D | Parfumöl | Fragrance (Parfum) | 0,3 | 0,3 | 0,3 | |
| | -(-Alpha-)-Bisabolol nat. | Bisabolol | 0,1 | 0,1 | 0,1 | |
| | | | | | | |
| | Angaben in Gew.-% | Total: | 100 | 100 | 100 | 100 |

Herstellungsvorschriften:

Beispiele F1/F2/F3: Jeweils Teil A: Auf ca. 85°C erhitzen. Teil B: Rohstoffe ohne Carbopol und Keltrol einwiegen. Carbopol und Keltrol mit Ultra Turrax eindispergieren. Auf ca. 85°C erhitzen. Teil B zu A geben. Teil C: Sofort zu A/B geben und sodann heiß homogenisieren (Ultra Turrax). Unter Rühren abkühlen lassen. Teil D: Zugeben und verrühren.

Beispiel F4: Teil A: Auf ca. 85°C erhitzen (ohne Keltrol und Zinkoxid). Keltrol und Zinkoxid mit dem Ultra Turrax in die heiße Lipidphase eindispergieren. Teil B: Auf ca. 85°C erhitzen. B zu A geben. Unter Rühren auf 60°C abkühlen und homogenisieren (Ultra Turrax). Anschließend unter Rühren auf Raumtemperatur (ca. 20°C) abkühlen lassen. Teil C: Zugeben und homogenisieren.

Die Beispiele F1-F4 sind als Standardrezepturen zu verstehen, da ebenso andere Sonnenfilter (allein oder als Sonnenfilterzusammensetzungen) von einer Kombination mit dem erfindungsgemäßen Emulgator profitieren.

Beispiel F5: Haargel-Wax for Men
Beispiel F6: Haarcreme (O/W)
Beispiel F7: Haarkurspülung (O/W)
Beispiel F8: Sensitiv Balsam Roll-on (O/W)
Beispiel F9: Pflegelotion für Feuchttücher (O/W)
Beispiel F10: Hautpflegecreme (O/W)

| | Rohstoff | INCI-Bezeichnung | F5 | F6 | F7 | F8 | F9 | F10 |
|---|---|---|---|---|---|---|---|---|
| A | Abil 350 | Dimethicone | | | | | | 1,5 |
| | Abil B 8852 | Dimethicone Copolyol | | 1 , 0 | | | | |
| | Cetiol HE | PEG-7 Glyceryl Cocoate | 1,0 | | | | | |
| | Cutina HR Plv. | Hydrogenated Castor Oil | | | 0,5 | | | |
| | Dracorin GMS | Glyceryl Stearate | | | 3,0 | 2,0 | | 1,0 |
| | Drago-Oat-Active | Water (Aqua), Butylene Glycol, Avena Sativa (Oat) Kernel Extr. | | | | | 1,0 | |
| | Dragoxat 89 | Ethylhexyl Isononanoate | | | | | | 7,0 |
| | Emulgator gemäß Beispiel 1A | | 15,0 | | 1,0 | | | |
| | Emulgator gemäß Beispiel 1 B | | | | | 0,5 | | 2,0 |
| | Eumulgin B2 | Ceteareth-20 | | | | 2,0 | | |
| | Farnesol | Farnesol | | 0,1 | | | | |
| | Fitoderm | Vegetable Squalane | | | | | | 3,0 |
| | Lanette 16 | Cetyl Alcohol | | | | 2,5 | | 4,0 |
| | Lanette 0 | Cetearyl Alcohol | | 4,0 | 1,5 | | | |
| | Lösungsvermittler | PEG-40 Hydrogenated Castor Oil, Trideceth-9, Water (Aqua) | 15,0 | | | | | |
| | Neo-Dragocid Flüssig | Triethylene Glycol, Imidazolidinyl Urea, Methylparaben, Propylparaben, Dehydroacetic Acid | | | | | 0,4 | |
| | Neutralöl | Caprylic/Capric Triglyceride | 10,0 | | | | | |
| | PCL Liquid 100 | Cetearyl Ethylhexanoate | 5,0 | 2,0 | 0,5 | 1,0 | | |
| | Pemulen TR-2 | Acrylates/C10-30 Alkyl Acrylate Crosspolymer | | | | | 0,2 | |
| | Rewoderm LI 520-70 | PEG-200 Hydrogenated Glyceryl Palmate | 1,5 | | | | | |
| | Varisoft BT 85 | Behentrimonium Chloride | | 1,0 | | | | |
| | Varisoft TA 100 | Distearyldimonium Chloride | | | 2,0 | | | |
| | Wasser | Water (Aqua) | | | | | 76,6 | |
| | | | | | | | | |
| B | -(-Alpha-) Bisabolol, nat. | Bisabolol | | | | | 0,1 | |

(fortgesetzt)

| | Rohstoff | INCI-Bezeichnung | F5 | F6 | F7 | F8 | F9 | F10 |
|---|---|---|---|---|---|---|---|---|
| | Aloe Vera-Gel-Konz.10/1 | Water (Aqua), Aloe Barbadensis Gel | | | | 1,0 | | |
| | Butylenglykol | Butylene Glycol | 1,0 | | | | | |
| | Citronensäure, 10%ig in Wasser | Citric Acid | 0,3 | | | | | |
| | Dragocid Liquid | Phenoxyethanol, Methylparaben, Ethylparaben, Butylparaben, Propylparaben, Isobutylparaben | | | 0,8 | 0,8 | | 0,8 |
| | Dragoxat 89 | Ethylhexyl Isononanoate | | | | | 8,0 | |
| | Emulgator gemäß Beispiel 1A | | | | | | 0,8 | |
| | Emulgator gemäß Beispiel 1B | | | 2,0 | | | | |
| | Glycerin | Glycerin | | 6,0 | | | | 3,0 |
| | Glydant Plus Liquid | DMDM Hydantoin, Iodopropynyl Butylcarbamate | 0,2 | | | | | |
| | Keltrol F | Xanthan Gum | | | | | | 0,25 |
| | Paraffinöl 5 Gr.E | Paraffinum Liquidum | | | | | 8,3 | |
| | PCL Liquid 100 | | | | | | 3,9 | |
| | Wasser | | 50,8 | 82,7 | 86,7 | 88,2 | | 77,15 |
| C | Deolite | Pentylene Glycol, Dimethyl Phenylpropanol | | | | 1,0 | | |
| | Dragocid Liquid | Phenoxyethanol, Methylparaben, Ethylparaben, Butylparaben, Propylparaben, Isobutylparaben | | 0,8 | | | | |
| | Dragoderm | Glycerin, Triticum Vulgare (Wheat) Gluten, Water Aqua | | | 3,5 | | | |
| | NaOH 10% Lsg, | Sodium Hydroxide | | | | | 0,4 | |
| | Parfümöl | Fragrance | 0,2 | 0,4 | 0,5 | 1,0 | | |
| | | | | | | | | |
| D | Parfümöl | Fragrance | | | | | 0,3 | 0,3 |
| | | | | | | | | |
| | Angaben in Gew.-% | Total: | 100 | 100 | 100 | 100 | 100 | 100 |

Herstellungshinweise:

Beispiel F5: Phasen A und B getrennt voneinander auf ca. 75°C erhitzen. Unter moderatem Rühren zusammengeben bis der Gel-Wax homogen ist. Anschließend abkühlen lassen, Phase C bei ca. 40°C zugeben und bis zur Homogenität einrühren. pH-Wert: ca. 5,2.

Beispiel F6: Alle Rohstoffe der Phase A mischen, auf 80°C erhitzen und mit einem Ultra Turrax homogenisieren. Mit einem Blattrührer kaltrühren, wobei die Rührgeschwindigkeit mit abnehmender Temperatur reduziert wird. Phase C bei ca. 35°C zugeben. pH-Wert: ca. 5,9.

Beispiele F7/F8: Jeweils Phasen A und B getrennt voneinander auf ca. 80°C erhitzen. Phase B zu A geben (Ultra-Turrax) und emulgieren. Mit einem Blattrührer kaltrühren, dabei die Rührgeschwindigkeit mit abnehmender Temperatur reduzieren. Phase C bei ca. 30°C zugeben. pH-Wert: ca. 4,2 für (F7) und 5,2 für (F8).

Beispiele F9/F10: Jeweils Pemulen TR-2 bzw. Keltrol F in Wasser unter einem Ultra-Turrax quellen. Phasen A und B getrennt voneinander auf ca. 80°C erhitzen. Phase B zu A geben (Ultra-Turrax) und emulgieren. Phase C zugeben und nochmals homogenisieren. Mit einem Blattrührer kaltrühren, dabei die Rührgeschwindigkeit mit abnehmender Temperatur reduzieren. Phase D bei ca. 35°C zugeben. pH-Wert: ca. 5,5 für (F9) und 5,2 für (F10).

Die obigen Beispiele F7, F8 und F9 sind Formulierungsbeispiele für niederviskose und sprühfähige Emulsionen. Ein weiteres Formulierungsbeispiel für eine niederviskose, nämlich sprühfähige Formulierung wird mit dem nachfolgenden Beispiel 11 gegeben.

Beispiel F11: Sprühfähige Sonnenmilch

| Rohstoffe | INCI-Bezeichnung | Gew.- % | Gew.- % |
|---|---|---|---|
| Phase A | | | |
| Wasser | Water (Aqua) | 73,60 | 73,60 |
| Pemulen TR-2 | Acrylates/C10-30 Alkyl Acrylate Crosspolymer | 0,20 | 0,20 |
| Neo-Dragocid Flüssig | Triethylen Glycol, Imidazolidinyl Urea, Methylparaben, Propylparaben, Dehydroacetic Acid | 0,40 | 0,40 |
| Drago-Oat-Active | Water (Aqua), Butylene Glycol, Avena Sativa (Oat) Kernel Extract | 1,00 | 1,00 |
| Phase B | | | |
| Emulgator gemäß Beispiel 1 A | | 0,80 | |
| Emulgator gemäß Beispiel 1 B | | | 0,80 |
| Dragoxat 89 | Ethylhexyl Isononanoate | 8,00 | 8,00 |
| Paraffinöl 5 Grad E | Paraffinum Liquidum | 8,30 | 8,30 |
| -(-Alpha-)Bisabolol, Natürlich | Bisabolol | 0,10 | 0,10 |
| Neo Heliopan A V | Ethylhexyl Methoxycinnamate | 3,00 | 3,00 |
| Neo Heliopan MBC | 4-Methylbenzylidene Camphor | 3,00 | 3,00 |
| Neo Heliopan 357 | Butyl Methoxybenzoylmethane | 0,90 | 0,90 |
| Phase C | | | |
| Natriumhydroxid 10 %-ige Lösung | Sodium Hydroxide | 0,40 | 0,40 |
| Phase D | | | |
| Parfümöl | Fragrance | 0,30 | 0,30 |

Formulierungsbeispiele für höherviskose und sehr feste Emulsionen ergeben sich aus den nachfolgenden Beispielen F12 und F13

Beispiel F12: Sonnenschutzcreme (O/W)

| Rohstoffe | INCI-Bezeichnung | Gew.- % | Gew.- % |
|---|---|---|---|
| Phase A | | | |
| Emulgator gemäß | | 2,00 | |
| Beispiel 1A | | | |
| Emulgator gemäß Beispiel 1B | | | 2,00 |
| Lanette O | Cetearyl Alcohol | 1,00 | 1,00 |
| Edenor L2 S.M. | Stearic Acid, Palmitic Acid | 4,00 | 4,00 |
| Neutralöl | Caprylic/Capric Acid | 10,00 | 10,00 |
| Dow Corning 200 Fluid 100 cS | Dimethicone | 0,30 | 0,30 |
| Neo Heliopan AV | Ethylhexyl Methoxycinnamate | 7,50 | 7,50 |
| Neo Heliopan BB | Benzophenone-3 | 4,50 | 4,50 |
| Neo Heliopan 357 | Butyl Methoxydibenzoylmethane | 2,00 | 2,00 |
| Phase B | | | |
| Wasser | Water (Aqua) | 66,86 | 66,86 |
| Carbopol 980 | Carbomer | 0,40 | 0,40 |
| Kaliumhydroxid 50 %ige wässr. Lösung | Potassium Hydroxide | 0,34 | 0,34 |
| Dragocid Liquid | Phenoxyethanol, Methylparaben, Ethylparaben, Butylparaben, Propylparaben, Isobutylparaben | 0,80 | 0,80 |
| Phase C | | | |
| Parfümöl | Fragrance | 0,30 | 0,30 |

Beispiel F13: Hautpflegecreme (O/W)

| Rohstoffe | INCI-Bezeichnung | Gew.-% | Gew.-% |
|---|---|---|---|
| Phase A | | | |
| Emulgator gemäß Beispiel 1A | | 2,00 | |
| Emulgator gemäß Beispiel 1B | | | 2,00 |
| Dragoxat 89 | Ethylhexyl Isononanoate | 7,00 | 7,00 |
| Lanette 16 | Cetyl Alcohol | 4,00 | 4,00 |
| Dracorin GMS | Glyceryl Stearate | 1,00 | 1,00 |
| Fitoderm | Vegetable Squalane | 3,00 | 3,00 |
| Abtil 350 | Dimethicone | 1,50 | 1,50 |
| Phase B | | | |
| Wasser | Water (Aqua) | 77,15 | 77,15 |
| Keltrol F | Xanthan Gum | 0,25 | 0,25 |
| Dragocid Liquid | Phenoxyethanol, Methylpara- | 0,80 | 0,80 |
| | ben, Ethylparaben, Butylparaben, Propylparaben, Isobutylparaben | | |
| Glycerin 85%ig in Wasser | Glycerin | 3,00 | 3,00 |

(fortgesetzt)

| Rohstoffe | INCI-Bezeichnung | Gew.-% | Gew.-% |
|---|---|---|---|
| Phase C | | | |
| Parfümöl | Fragrance | 0,30 | 0,30 |

Beispiel F14: Sonnenschutzmilch mit hohem Sonnenschutzfaktor

| Rohstoffe | INCI-Bezeichnung | Gew.-% | Gew.-% |
|---|---|---|---|
| Phase A | | | |
| Emulgator gemäß Beispiel 1A | | 2,00 | |
| Emulgator gemäß Beispiel 1B | | | 2,20 |
| Parsol® SLX | Poylsilicone-15 | 6,00 | 6,00 |
| Neo Heliopan® 357 | Butyl Methoxydibenzoylmethan | 2,00 | 2,00 |
| Parsol® 5000 | 4-Methylbenzylidene Camphor | 4,00 | 4,00 |
| Uvinul® T150 | Ethylhexyltriazone | 2,00 | 2,00 |
| Silicone DC 200/350 cs | Dimethicone | 1,00 | 1,00 |
| Lanette O | Cetearyl Alcohol | 2,00 | 2,00 |
| Softisan 100 | Hydrogenated Coco-Glycerides | 3,00 | 3,00 |
| Tegosoft TN | C12-15 Alkyl Benzoate | 6,00 | 6,00 |
| Cetiol B | Dibutyl Adipate | 7,00 | 7,00 |
| Vitamin E acetate | Tocopheryl Acetate | 2,00 | 2,00 |
| BHT | BHT | 0,05 | 0,05 |
| Edeta BD | Disodium EDTA | 0,10 | 0,10 |
| Phenonip | Phenoxyethanol, Methylparaben, Ethylparaben, Propylparaben Butylparaben | 0,60 | 0,60 |
| Phase B | | | |
| Wasser | Water (Aqua) | Ad 100 | Ad 100 |
| 1,2-Propylenglykol | Propylene Glycol | 5,00 | 5,00 |
| Carbopol 980 | Carbomer | 0,30 | 0,30 |
| KOH, 10%ige wässrige Lösung | Potassium Hydroxide | 1,50 | 1,50 |
| Phase C | | | |
| Verkapselter UV-Filter Eusolex® UV-Pearls™ OMC | Ethylhexyl Methoxycinnamate | 12,00 | 18,00 |
| Phase D | | | |
| Parfümöl | Fragrance | 0,30 | 0,30 |

Testmethode 211 zur Bestimmung der Verseifungszahl

Ca. 1,5 g der Probe werden genau gewogen und mit 25,00 ml 0,5 N ethanolischer Kalilauge versetzt. Nach Zugabe eines Siedesteines wird das Gemisch 1 Stunde lang unter Rückflusskühlung am Sieden gehalten. Anschließend wird der Laugenüberschuss in dem noch warmen Gemisch nach Zugabe einiger Tropfen einer 0,1 %igen ethanolischen Thymolphthalein-Lösung bei 40°C unter möglichst intensivem Rühren mit 0,5 N Schwefelsäure bis zum Indikatorumschlag titriert. Nach nochmaligem Zusatz von wenig Thymolphthalein-Lösung wird 2 Minuten lang bei 40°C weitergerührt. Die sich evtl. wieder färbende Flüssigkeit wird nochmals mit 0,5 N Schwefelsäure bis zur Entfärbung titriert.

In gleicher Weise ist ein Blindversuch anzusetzen. Aus der Differenz der im Blindversuch und Hauptversuch verbrauchten 0,5 N Schwefelsäure wird die Verseifungszahl berechnet.

Berechnung:

$$\text{Säurezahl} = \frac{(B - H) * 28}{w}$$

B = Milliliter der im Blindversuch verbrauchten 0,5 N Schwefelsäure
H = Milliliter der im Hauptversuch verbrauchten 0,5 N Schwefelsäure
w = Substanzeinwaage der Probe in Gramm.

Testmethode 228 zur Bestimmung der Säurezahl

Ca. 2,5 g der zu untersuchenden Substanz werden genau in einen 300 ml Weithalserlenmeyerkolben eingewogen, mit 50 ml eines Gemisches aus gleichen Volumenteilen Diethylether/Ethanol/Wasser (gegen Phenolphthalein neutralisiert) versetzt und unter gelindem Erwärmen auf dem Wasserbad (max. 35°C) weitgehend in Lösung gebracht. Der Erlenmeyerkolben wird dabei mit einem Uhrglas abgedeckt. Eine vollständige Lösung der Substanz erfolgt dabei nicht, es resultiert lediglich eine trübe Dispersion. Sofort nach dem Lösen wird mit einer 0,1 N KOH-Lösung bis zum ersten Auftreten einer schwachen Rosafärbung titriert.

Berechnung:

$$\text{Säurezahl} = \frac{a * 5,61}{w}$$

a = Anzahl der verbrauchten Milliliter 0,1 N Kaliumhydroxid-Lösung
w = Substanzeinwaage in Gramm

**Patentansprüche**

1. O/W-Emulgator, umfassend

    (a) 30 - 50 Gew.-% gehärtete Palmölglyceride,
    (b) 15 - 35 Gew.-% Kalium-Cetylphosphate,
    (c) 20 - 30 Gew.-% Cetylalkohol, und
    (d) 5 - 15 Gew.-% Kaliumphosphate,

wobei sich die Gewichtsprozentangaben auf die Gesamtmasse des O/W-Emulgators beziehen.

2. O/W-Emulgator nach Anspruch 1,
wobei der Emulgator kein Polyethylenglykol (PEG) und/oder kein sonstiges Glykol und/oder kein Paraffin und/oder kein Isoparaffin enthält.

3. O/W-Emulgator nach einem der vorangehenden Ansprüche, bestehend aus:

    (a) 35 - 45 Gew.-% gehärteten Palmölglyceriden,

(b) 15 - 35 Gew.-% Kalium-Cetylphosphaten,
(c) 25 - 30 Gew.-% Cetylalkohol,
(d) 5 - 10 Gew.-% Kaliumphosphaten,
(e) 0 - 15 Gew.-% sonstigen Stoffen,

wobei sich die Gewichtsprozentangaben auf die Gesamtmasse des O/W-Emulgators beziehen.

**4.** O/W-Emulgator nach einem der vorangehenden Ansprüche, wobei der Bestandteil (b) Kalium-Monocetylphosphat und Kalium-Dicetylphosphat enthält, vorzugsweise in einem Gewichtsverhältnis von 2,5 : 1 oder größer, bevorzugt in einem Gewichtsverhältnis im Bereich von 10 : 1 bis 2,5 : 1, weiter bevorzugt im Bereich von 6 : 1 bis 3 : 1.

**5.** O/W-Emulgator nach einem der vorangehenden Ansprüche, wobei der Bestandteil (d) Kaliumhydrogenphosphat enthält, und vorzugsweise der Anteil an Kaliumhydrogenphosphat bei 4 Gew.-% oder höher liegt, bezogen auf die Gesamtmasse des O/W-Emulgators.

**6.** O/W-Emulgator nach einem der vorangehenden Ansprüche, wobei der O/W-Emulgator in Pastillenform vorliegt.

**7.** O/W-Emulsion, umfassend:

- eine Wasserphase
- eine in der Wasserphase dispergierte Ölphase und
- 0,25-15 Gew.-%, vorzugsweise 1-15 Gew.-% eines O/W-Emulgators nach einem der vorangehenden Ansprüche, wobei die Gewichtsprozentangabe auf die Gesamtmasse der O/W-Emulsion bezogen ist.

**8.** O/W-Emulsion nach Anspruch 7, zusätzlich umfassend

- 0,1-10 Gew.-% eines Stabilisierungsmittels und/oder
- 1-10 Gew.-% eines Co-Emulgators,

wobei die Gewichtsprozentangabe auf die Gesamtmasse der O/W-Emulsion bezogen ist.

**9.** O/W-Emulsion nach Anspruch 7 oder 8, wobei der pH der Emulsion auf einen Wert zwischen 3 und 11, vorzugsweise zwischen 4 und 9, weiter vorzugsweise zwischen 4 und 7 eingestellt ist.

**10.** O/W-Emulsion nach einem der Ansprüche 7-9, weiter umfassend:

- dispergierte Feststoffe
und/oder
- UV-A- und/oder UV-B-Filter
und/oder
- ein Antioxidationsmittel,
und/oder
- Parfümöle
und/oder
- sonstige Hilfsstoffe

**11.** Verfahren zur Herstellung einer O/W-Emulsion nach einem der Ansprüche 7-10, mit folgenden Schritten:

- Bereitstellen einer Wasser- und einer Ölphase
- Bereitstellen eines Emulgators nach einem der Ansprüche 1-6,
- Einarbeiten des Emulgators in die Wasser- und/oder in die Ölphase;
- Nach Einarbeiten des Emulgators Vermischen der Wasser- mit der Ölphase, bis die O/W-Emulsion gebildet ist.

**Claims**

**1.** An O/W emulsifier, comprising

(a) 30 - 50 wt.% hydrogenated palm oil glycerides,
(b) 15 - 35 wt.% potassium cetyl phosphates,
(c) 20 - 30 wt.% cetyl alcohol and
(d) 5 - 15 wt.% potassium phosphates,

wherein the percentages by weight indicated relate to the total mass of the O/W emulsifier.

2. The O/W emulsifier according to claim 1,
wherein the emulsifier contains no polyethylene glycol (PEG) and/or no other glycol and/or no paraffin and/or no isoparaffin.

3. The O/W emulsifier according to one of the preceding claims, consisting of:

(a) 35 - 45 wt.% hydrogenated palm oil glycerides,
(b) 15 - 35 wt.% potassium cetyl phosphates,
(c) 25 - 30 wt.% cetyl alcohol,
(d) 5 - 10 wt.% potassium phosphates,
(e) 0 - 15 wt.% other substances,

wherein the percentages by weight indicated relate to the total mass of the O/W emulsifier.

4. The O/W emulsifier according to one of the preceding claims, wherein the component (b) contains potassium monocetyl phosphate and potassium dicetyl phosphate, preferably in a ratio by weight of 2.5 : 1 or greater, for preference in a ratio by weight in the range of 10 : 1 to 2.5 : 1 and more preferably in the range of 6 : 1 to 3 : 1.

5. The O/W emulsifier according to one of the preceding claims, wherein the component (d) contains potassium hydrogen phosphate and preferably the proportion of potassium hydrogen phosphate is 4 wt.% or higher, based on the total mass of the O/W emulsifier.

6. The O/W emulsifier according to one of the preceding claims, wherein the O/W emulsifier is in the form of pastilles.

7. An O/W emulsion, comprising:

- an aqueous phase
- an oil phase dispersed in the aqueous phase and
- 0.25 - 15 wt.%, preferably 1 - 15 wt.%, of an O/W emulsifier according to one of the preceding claims, wherein the percentage by weight indicated relates to the total mass of the O/W emulsion.

8. The O/W emulsion according to claim 7, also comprising:

- 0.1 - 10 wt.% of a stabiliser and/or
- 1 - 10 wt.% of a co-emulsifier;

wherein the percentage by weight indicated relates to the total mass of the O/W emulsion.

9. The O/W emulsion according to claim 7 or 8, wherein the pH of the emulsion is adjusted to a value of between 3 and 11, preferably between 4 and 9 and more preferably between 4 and 7.

10. The O/W emulsion according to one of claims 7 to 9, also comprising:

- dispersed solids
and/or
- UV-A and/or UV-B filters
and/or
- an antioxidant
and/or
- perfume oils
and/or

- other auxiliary substances.

11. A process for the production of an O/W emulsion according to one of claims 7 to 10, comprising the following steps:

- preparing an aqueous phase and an oil phase
- preparing an emulsifier according to one of claims 1 to 6,
- incorporating the emulsifier into the aqueous phase and/or the oil phase;
- after incorporation of the emulsifier, mixing the aqueous phase with the oil phase until the O/W emulsion has formed.

**Revendications**

1. Emulsifiant huile dans l'eau, comprenant

(a) 30 à 50% en poids de glycérides d'huile de palme durcies
(b) 15 à 35% en poids de cétylphosphates de potassium
(c) 20 à 30% en poids d'alcool cétylique ; et
(d) 5 à 15% en poids de phosphates de potassium.

les indications des pourcentages en poids se référant à la masse globale de l'émulsifiant huile dans l'eau.

2. Emulsifiant huile dans l'eau selon la revendication 1,
dans lequel l'émulsifiant ne comprend pas de polyéthylèneglycol (PEG), et/ou pas d'autre glycol et/ou pas de paraffine et/ou pas d'isoparaffine.

3. Emulsifiant huile dans l'eau selon l'une des revendications précédentes, comprenant

(a) 35 à 45% en poids de glycérides d'huile de palme durcies ;
(b) 15 à 35% en poids de cétylphosphates de potassium ;
(c) 25 à 30% en poids d'alcool cétylique ;
(d) 5 à 10% en poids de phosphates de potassium ;
(e) 0 à 15% en poids d'autres substances ;

les indications des pourcentages en poids se référant à la masse globale de l'émulsifiant huile dans l'eau.

4. Emulsifiant huile dans l'eau selon l'une des revendications précédentes, dans lequel le composant (b) contient du monocétylphosphate de potassium et du dicétylphosphate de potassium, de préférence dans un rapport en poids de 2,5 : 1 ou plus, dans les cas plus préférés dans un rapport en poids compris dans l'intervalle allant de 10 : 1 à 2,5 : 1, dans les cas encore plus préférés dans un intervalle allant de 6 : 1 à 3 : 1.

5. Emulsifiant huile dans l'eau selon l'une des revendications précédentes, dans lequel le composant (d) contient du hydrogénophosphate de potassium, la teneur en hydrogénophosphate de potassium représentant de préférence 4% en poids ou plus, par rapport à la masse globale de l'émulsifiant huile dans l'eau.

6. Emulsifiant huile dans l'eau selon l'une des revendications précédentes, dans lequel l'émulsifiant huile dans l'eau se présente sous forme de pastilles.

7. Emulsion huile dans l'eau, comprenant :

- une phase aqueuse ;
- une phase huileuse dispersée dans la phase aqueuse ; et
- 0,25 à 15 % en poids, de préférence 1 à 15% en poids d'un émulsifiant huile dans l'eau selon l'une des revendications précédentes, l'indication du pourcentage en poids se référant à la masse globale de l'émulsion huile dans l'eau.

8. Emulsion huile dans l'eau selon la revendication 7, comprenant en outre :

- 0,1 à 10% en poids d'un stabilisateur ; et/ou
- 1 à 10% en poids d'un co-émulsifiant;

l'indiction du pourcentage en poids se référant à la masse globale de l'émulsion huile dans l'eau.

9. Emulsion huile dans l'eau selon les revendications 7 ou 8, dans lequel le pH de l'émulsion est ajusté à une valeur comprise entre 3 et 11, dans les cas plus préférés entre 4 et 9, et dans les cas encore plus préférés entre 4 et 7.

10. Emulsion huile dans l'eau selon l'une des revendications 7 à 9, comprenant en outre :

- des solides dispersés
et/ou
- un filtre UV-A et/ou UV-B
et/ou
- un antioxydant ;
et/ou
- des huiles de parfum
et/ou
- d'autres adjuvants.

11. Procédé de production d'une émulsion huile dans l'eau selon l'une des revendications 7 à 10, comprenant les étapes ci-dessous :

- fourniture d'une phase aqueuse et d'une phase huileuse ;
- fourniture d'un émulsifiant selon l'une des revendications 1 à 6 ;
- incorporation de l'émulsifiant dans la phase aqueuse et/ou dans la phase huileuse ;
- après l'incorporation de l'émulsifiant, mélange de la phase aqueuse avec la phase huileuse jusqu'à la formation de l'émulsion huile dans l'eau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1264632 A
- EP 0227012 A
- EP 0251249 A
- EP 0427411 A
- WO 2004075868 A
- DE 10055940
- WO 0238537 A
- EP 0900781 A
- EP 1029849 A
- EP 1066821 A
- WO 0143712 A
- WO 0170176 A
- WO 0198235 A
- WO 0198258 A